Europäisches Patentamt

European Patent Office

Office européen des brevets

(11) Numéro de publication : **0 438 358 B1**

(12) **FASCICULE DE BREVET EUROPEEN**

(45) Date de publication du fascicule du brevet :
**27.04.94 Bulletin 94/17**

(51) Int. Cl.⁵ : **A61K 9/16, B01J 2/04**

(21) Numéro de dépôt : **91400105.2**

(22) Date de dépôt : **18.01.91**

(54) **Nouvelle forme unitaire, solide et poreuse comprenant des particules sous forme de perles ainsi que sa préparation.**

(30) Priorité : **19.01.90 FR 9000624**

(43) Date de publication de la demande :
**24.07.91 Bulletin 91/30**

(45) Mention de la délivrance du brevet :
**27.04.94 Bulletin 94/17**

(84) Etats contractants désignés :
**AT BE CH DE DK ES FR GB GR IT LI LU NL SE**

(56) Documents cités :
**EP-A- 0 277 508
EP-A- 0 352 190
FR-A- 2 613 223
US-A- 3 433 872
US-A- 4 389 356**

(73) Titulaire : **FARMALYOC
5 Avenue Charles De Martigny
F-94701 Maisons Alfort (FR)**

(72) Inventeur : **Coutel, Anne
3, avenue de la Résidence
F-92160 Antony (FR)**
Inventeur : **Lebreton, Guy
21, Résidence de Courcelle
F-91190 Gif-sur-Yvette (FR)**
Inventeur : **Veillard, Michel
12, rue du Docteur Roux
F-92330 Sceaux (FR)**

(74) Mandataire : **Savina, Jacques et al
RHONE-POULENC RORER S.A., Direction des
Brevets, 90 avenue Raymond Aron
F-92165 Antony Cédex (FR)**

## Description

La présente invention concerne une forme lyophilisée unitaire, solide et poreuse, comprenant des particules sous forme de perles ainsi que son procédé de préparation.

La préparation de microparticules est principalement utilisée de manière à retarder la dissolution des principes actifs et trouve, de ce fait, de nombreuses applications dans le domaine des médicaments à libération contrôlée ainsi que dans le domaine du masquage du goût des médicaments destinés à l'administration orale. Il a néanmoins toujours été difficile de mettre au point une formulation contenant des microparticules sous forme de doses unitaires et notamment une formulation convenable à une administration orale.

En effet, la préparation industrielle de comprimés et de gélules nécessite des qualités d'écoulement et/ou de cohésion du granulé à diviser que ne possèdent pas nécessairement les microparticules.

Le comprimé pose des problèmes d'intégrité des microparticules sous l'effet de la compression ; sa vitesse de désagrégation lente ne permet pas toujours de l'administrer après désagrégation et mise en suspension dans un verre d'eau.

La gélule ne permet pas toujours la dispersion des particules dans le tractus gastrointestinal.

De plus, la gélule ainsi que le comprimé posent des problèmes de déglutition particulièrement marqués chez l'enfant et chez le vieillard.

Enfin, le sachet est une forme pharmaceutique coûteuse, d'utilisation non ambulatoire et la forme sirop sec n'est pas souvent réalisable pour des raisons de libération précoce du principe actif hors des microparticules ou de stabilité physique et/ou chimique de la préparation.

La fabrication de perles, mieux connue dans l'art antérieur sous le terme de prills, est décrite dans les brevets EP 277 508, US 4 525 198, US 4 389 356. Les procédés de préparation de ces perles ne sont malheureusement pas applicables à tous les produits.

Ces procédés sont utilisés pour des produits pouvant être soumis à un chauffage violent destiné à les faire fondre et à un refroidissement rapide pour assurer leur solidification. En revanche, ils sont mal adaptés à l'industrie pharmaceutique où l'on désire des dimensions de perles régulières et de l'ordre de quelques dizaines à quelques centaines de microns.

Les brevets français 2 036 890 et 2 366 835 décrivent des formes pharmaceutiques ayant pour caractéristique de se dissoudre ou de se désintégrer rapidement en milieu aqueux ou dans la salive.

La demande de brevet EP 352 190 décrit des lyophilisats convenables pour un usage ambulatoire et contenant des micro (ou nano)particules préparées par des méthodes connues.

La demande de brevet français 2 613 223 décrit une forme galénique sous forme de grains hydrosolubles.

Il a été maintenant trouvé que des techniques ayant des objectifs totalement opposés, comme le retard de la dissolution d'un principe actif d'une part et le fait que la forme pharmaceutique se désagrège ou se dissolve rapidement, d'autre part, pouvaient être associées de manière à obtenir une forme unitaire lyophilisée, facilement et rapidement désintégrable dans l'eau, contenant le principe actif sous forme de perles, et dans laquelle la décantation et/ou la remontée en surface des particules au cours de la lyophilisation a été évitée.

Selon l'invention, le procédé de préparation de la forme unitaire, solide consiste à

1) préparer un mélange de perles contenant une quantité prédéterminée d'un ou plusieurs principes actifs,

2) incorporer le mélange obtenu dans une pâte destinée à être lyophilisée et contenant

    a) au moins une substance choisie parmi

        - des matières épaississantes et structurantes servant de support et

        - des ballasts,

    b) un ou plusieurs agents stabilisants empêchant la décantation et/ou la remontée en surface des perles dans les dans le mélange,

    c) éventuellement un ou plusieurs autres principes actifs ou mélanges de perles contenant un principe actif,

    d) une quantité d'eau convenable de manière à ajuster la viscosité de la composition,

3) lyophiliser la pâte obtenue.

Le produit lyophilisé obtenu peut être divisé mécaniquement en doses unitaires présentant une forme et un volume bien définis, mais il est préférable de répartir la pâte dans des alvéoles de forme et de dimension prédéterminées, préalablement à l'opération de lyophilisation. Le dosage du/des principe(s) actif(s) dans la pâte et la forme et les dimensions des alvéoles étant calculés de manière à obtenir une quantité précisément définie du/des principe(s) actif(s) dans chaque dose unitaire.

Dans la description ci-dessus, ainsi que dans ce qui suivra, on entend par "principe actif" toute substance contenant au moins un produit thérapeutiquement actif, un agent de nutrition, un agent de diagnostic ou un agent cosmétique, celui-ci pouvant être en association avec une ou plusieurs autres substances du même type ou en association avec d'autres mélanges de perles contenant elles-mêmes d'autres substances citées ci-dessus.

Le mélange de perles contenant une quantité prédéterminée de principe actif est préparé par une méthode originale évitant d'avoir recours à un refroidissement très intense ou à des tours de hauteur trop importante.

En effet, certains principes actifs présentent lors de leur fusion des phénomènes dits de "surfusion" qui retardent considérablement leur solidification même après refroidissement. Il est maintenant possible d'effectuer la mise sous forme de billes régulières, quel que soit le principe actif, y compris dans le cas des principes actifs qui présentent un point de cristallisation non défini (ou plus communément un phénomène de surfusion) et qui sont donc peu favorables à la technique du "prilling" car ils ont tendance à rester sous forme huileuse ou pâteuse bien après un retour à une température inférieure à leur température de fusion.

La méthode de préparation des perles est caractérisée en ce que l'on mélange le principe actif avec un ou plusieurs excipients sous forme fondue, on force le passage de la masse fondue à travers une buse qui subit une vibration, on laisse tomber dans une tour à contre courant avec un gaz les perles formées, on rassemble les perles solides dans le bas de la tour.

A cette tour de prilling est éventuellement adjoint un lit fluidisé qui permet de maintenir en fluidisation permanente les perles non encore entièrement solidifiées.

Les additifs qui permettent d'induire la cristallisation du produit sont choisis parmi un ou plusieurs produits inertes vis à vis dudit principe actif, notamment:

- les alcools gras tels que :
  . l'alcool cétylique,
  . l'alcool stéarilique,
- les acides gras tels que :
  . l'acide stéarique,
  . l'acide palmitique,
- les esters de glycérol tels que :
  . le palmitostéarate de glycérol,
  . le stéarate de glycérol commercialisé sous la marque PRECIROL,
  . le béhénate de glycérol commercialisé sous la marque COMPRITOL,
- les huiles hydrogénées telles que :
  . l'huile de ricin hydrogénée commercialisée sous la marque CUTINA HR,
- les sels d'acides gras tels que :
  . le stéarate de magnésium ou de calcium,
- les polyols tels que :
  . le mannitol,
  . le sorbitol,
  . le xylitol,
- les cires telles que :
  . la cire blanche,
  . la cire de Carnauba,
  . la paraffine,
- les polyoxyéthylène glycols de poids moléculaire élevé,
- les polyoxyéthylène estérifiés tels que :
  . le distéarate de PEG-32,
  . le distéarate de PEG-150.

A ces additifs de cristallisation, il est parfois souhaitable d'adjoindre des polymères solubles ou dispersibles dans la masse fondue qui vont permettre une dissolution maîtrisée et modulable des perles lors de leur utilisation tels que :

- les dérivés cellulosiques (hydroxypropylcellulose, hydroxypropylméthylcellulose, hydroxyéthylcellulose, éthylcellulose, carboxyméthylcellulose),
- les résines acryliques (commercialisées sous la marque EUDRAGIT),
- les polyvinylacétates (commercialisées sous la marque RHODOPAS),
- les résines de polyalkylène (éthylène propylène), de polylactique, d'anhydride maléique, de silicone.

Certains additifs minéraux permettent, avec les additifs de cristallisation d'accélérer la solidification des principes actifs en particulier lorsque ces derniers présentent un phénomène de surfusion. On peut citer à titre d'exemple :

- les silices,
- les oxydes minéraux tels que l'oxyde de titane, de fer,
- les phosphates,
- les carbonates,

3

- les argiles,
- le talc.

Afin d'améliorer la dispersion du principe actif dans l'additif de cristallisation, il est parfois utile d'ajouter un agent tensioactif choisi par exemple parmi les esters de sorbitol, les polysorbates de polyoxyéthylène commercialisés sous la marque TWEEN, les glycols tels que la glycérine ou le propylène glycol.

Le procédé de préparation des perles consiste à préparer une masse fondue du/ou des principes actifs avec un ou plusieurs excipients. Cette masse peut être préparée par fusion séparée des divers constituants suivie de leur mélange ou par fusion du mélange des constituants. Les éventuels composés non solubles étant additionnés à la fin de la fusion de façon à obtenir une masse homogène.

La nature des constituants de la masse fondue est choisie par l'homme de l'art de la technique considérée en fonction de la compatibilité des constituants, de la viscosité du mélange des constituants, du diamètre de la buse, de l'hydrophilie du principe actif, de la tension superficielle du principe actif, de la taille particulaire des additifs insolubles, du débit de la buse, de la température de la tour, de sa hauteur et surtout de la dimension des perles désirées, du taux de principe actif à y inclure et du temps de libération du principe actif désiré.

La libération "in vitro" et la disponibilité "in vivo" du principe actif à partir de ces perles est modifiée (prolongée, retardée ou différée) grâce à l'additif de cristallisation qui selon sa nature permet une libération du principe actif en des temps deux à vingt fois plus longs que le même principe actif conditionné de façon classique par exemple sous forme de comprimés à libération immédiate. Ainsi, ces perles permettent une prise des médicaments préparés à partir de celles-ci quotidienne au lieu des 2 ou 3 prises quotidiennes avec le médicament classique à libération immédiate.

Parmi les additifs de cristallisation permettant un effet retard, on peut citer non limitativement les acides gras, les esters de glycérol, les huiles hydrogénées, les cires et les polyoxyéthylèneglycols estérifiées.

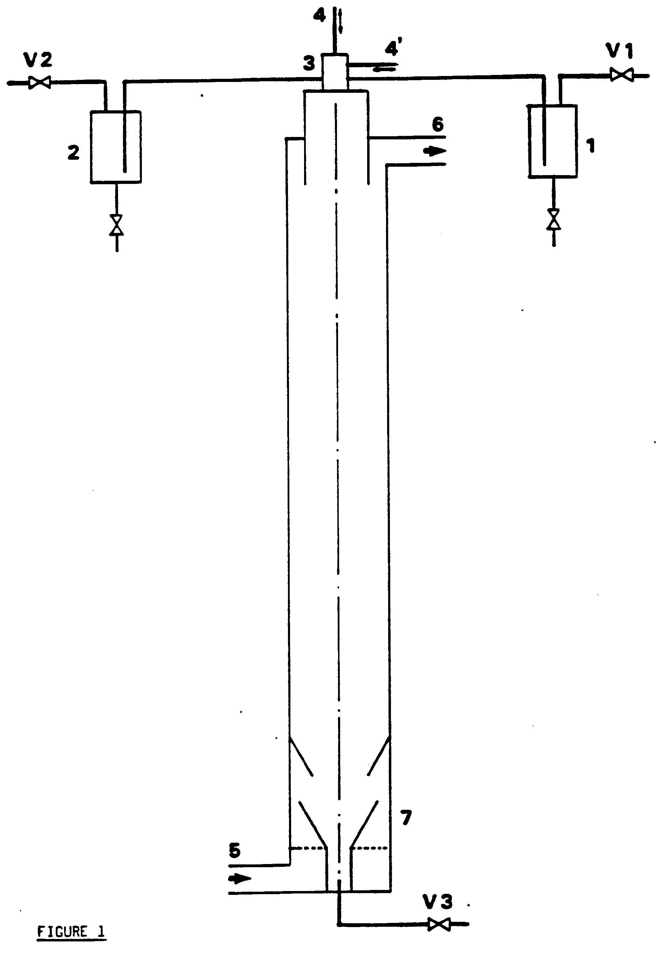

L'appareillage est plus complètement décrit à l'aide de la figure 1 ci-annexée. Le principe actif est introduit dans le récipient 1 et l'excipient dans le récipient 2 ou bien on introduit le mélange fondu, excipient plus principe actif dans chacun des deux récipients. Ces deux récipients sont maintenus sous atmosphère de gaz inerte. Au moyen de deux tubes, les liquides fondus sont amenés au-dessus d'une buse qui est maintenue dans une atmosphère non refroidie et qui est même éventuellement chauffée (3). La buse présente 1 à 24 perforations ou plus présentant de préférence un diamètre compris entre 50 et 600 microns. La longueur de la perforation est de préférence comprise entre 0,5 et 10 fois le diamètre de celle-ci.

Cette buse est soumise à un système de vibration (4) électrique de haute fréquence (500 à 10000 hertz). L'air froid qui permet la solidification du principe actif et de l'excipient est introduit en bas de tour (5) et sort en-dessous de la buse (6) à une distance de préférence d'environ L/10 par rapport au sommet de la tour, L étant la hauteur de la tour.

Chaque orifice de la buse a de préférence une forme de cône inversé, la pointe du cône se trouvant dirigée vers la base de la tour ce qui permet d'obtenir un flux de liquide parfaitement laminaire.

La hauteur de la tour varie entre 1 mètre et une dixaine de mètres, la tour peut comporter au quart inférieur de sa hauteur une jupe perforée tronconique qui centre les perles dans le lit fluidisé.

Le lit fluidisé (7) éventuellement adjoint au bas de la tour est dans le cadre de la présente invention de préférence un lit fluidisé en forme d'entonnoir équipé à sa base d'une grille de distribution permettant de minimiser l'adhérence sur les parois et de favoriser les chocs paroi-perles dans le but d'accroître la vitesse de solidification. L'adjonction de ce type d'appareil permet une solidification plus intense du mélange des constituants dans la bille, l'extérieur étant déjà solide à l'entrée dans le lit fluidisé.

Les billes ou perles obtenues par ce procédé présentent une forme régulière et un diamètre compris entre 0,1 mm et 1,5 mm. La quantité de principe actif introduite varie de 5 à 95 % en poids et de préférence de 40 à 60 % en poids.

La matière épaississante et structurante servant de support dans la préparation de la nouvelle forme unitaire peut être choisie parmi toute matière soluble ou dispersible dans l'eau, permettant d'assurer la cohésion de la masse, le cas échéant acceptable du point de vue pharmaceutique et inerte vis-à-vis du principe actif. Ces matières sont notamment choisies parmi les polypeptides comme la gélatine ou la gélatine partiellement hydrolysée, des colloïdes, des polysaccharides à poids moléculaire élevé, des hauts polymères pouvant donner des solutions colloïdales, par exemple des gommes naturelles (gomme arabique, gomme adragante...), des gommes synthétiques ou hémisynthétiques (glycosylglucanes, gomme xanthane...), le dextrane, la dextrine, les alginates (alginate de sodium), les pectinates, les dérivés de la cellulose (cellulose microcristalline, carboxyméthylcellulose...), les dérivés hydrodispersibles de l'amidon, les silices colloïdales, les bentonites, ou encore d'autres matières support telles que l'alcool polyvinylique, la polyvinylpyrrolidone, les polyéthylèneglycols (PEG 20 000, PEG 6 000 notamment), des polymères acryliques ou des copolymères, ou encore des mélanges de matières telles que citées ci-dessus.

De préférence, on utilise une matière hydrosoluble

On désigne par "ballasts" des matières de préférence solubles et cristallisables et le cas échéant pharmaceutiquement acceptables qui améliorent les propriétés physiques de la nouvelle forme unitaire. Ces substances peuvent être notamment choisies parmi le lactose, le glycocolle, le sorbitol, le mannitol, le glucose, les maltodextrines ou encore, éventuellement parmi des oxydes (oxyde de magnésium), des carbonates (carbonate de calcium), des phosphates (phosphate tricalcique), ou la cellulose micro-cristalline, ou des mélanges de ces substances.

Il est entendu que la pâte destinée à la lyophilisation doit nécessairement contenir au moins une substance choisie parmi les matières épaississantes et les ballasts cités ci-dessus, mais il est également avantageux de faire intervenir à la fois une ou plusieurs matières épaississantes et un ou plusieurs ballasts.

Les matières épaississantes et structurantes et les ballasts sont choisis de manière à conférer à la pâte destinée à la lyophilisation, un comportement rhéologique et une viscosité adaptés à une bonne division du produit et à la tenue en suspension des divers composants (écoulement, homogénéité, régularité du volume divisé, stabilité de la suspension pendant la division). Ces substances sont également choisies de manière à assurer la texture du produit final lyophilisé (par exemple dureté suffisante pour permettre l'extrusion à travers un blister).

La pâte destinée à la lyophilisation doit, en outre, contenir un ou plusieurs agents de suspension destinés à empêcher la sédimentation des particules pendant la lyophilisation. Ces agents stabilisants sont des substances particulaires solides de taille micronique, inertes vis-à-vis du mélange. Elles peuvent être soit insolubles, soit solubles et en excès par rapport au pouvoir solubilisant de la pâte à lyophiliser. Elles sont choisies de telle manière que leur vitesse de sédimentation soit du même ordre de grandeur que celles des perles. Le choix de l'agent stabilisant est de ce fait adapté en fonction de la densité et de la dimension des perles ainsi que des conditions de pH de la solution dans laquelle il est introduit. Parmi les agents stabilisants convenables peuvent être cités des oxydes (oxyde de titane, oxyde de magnésium, oxydes de fer, silice par exemple), des sels comme des carbonates (carbonate de calcium, carbonate de magnésium par exemple), des silicates (kaolin par exemple), des phosphates (phosphate tricalcique par exemple), des sucres (lactose, glucose, mannitol, lévulose, maltodextrine par exemple).

Dans le procédé selon l'invention, il est entendu que l'ordre d'introduction des différentes substances dépend de ces substances elles-mêmes, certaines d'entre elles pouvant être mélangées préalablement.

D'une manière générale, les matières épaississantes et structurantes constituent de 0,01 à 99 % en poids par rapport à la masse sèche du lyophilisat. Il est également possible de n'introduire que des ballasts et de ne pas employer de matière épaississante.

Les ballasts sont généralement en excès par rapport au pouvoir solubilisant de la pâte à lyophiliser. Ils constituent 1 à 99 % en poids par rapport à la masse sèche totale du lyophilisat. Il est cependant possible de n'introduire que la matière épaississante, sans employer de ballast.

L'agent stabilisant est fonction des particules qui constitueront la pâte à lyophiliser et représente de 1 à 70 % en poids par rapport à la masse sèche lyophilisée.

La quantité d'eau introduite est déterminée de telle manière que la pâte à lyophiliser ait un comportement rhéologique et une viscosité adaptés. Il est en effet souhaitable d'ajuster la viscosité de la pâte à lyophiliser de telle sorte qu'elle soit suffisamment fluide pour permettre une division régulière, et suffisamment visqueuse pour éviter la sédimentation des particules.

La quantité d'eau introduite dans la constitution du mélange pourra représenter 10 à 90 % en poids par rapport à la masse humide à lyophiliser, en fonction de la nature des substances choisies pour constituer le mélange.

Enfin, le mélange de perles contenant le/les principe(s) actif(s) est préparé de telle manière que le polymère ou la substance macromoléculaire introduite représente 0,1 à 80 % en poids par rapport à la masse sèche du lyophilisat.

En outre, la préparation destinée à être lyophilisée peut contenir éventuellement d'autres additifs comme par exemple des agents de surface, ou d'autres substances compatibles et le cas échéant pharmaceutiquement acceptables telles que des colorants, des substances édulcorantes ou modifiant le goût, des agents conservateurs, ou toute autre substance compatible avec le reste du mélange.

A titre d'exemple, les agents de surface peuvent être choisis parmi les agents non-ioniques [polysorbates polyoxyéthyléniques (Tween), esters de sorbitane (Span), copolymères d'oxyde d'éthylène et de propylène, éthers de polyoxyéthylèneglycols et d'alcool gras ...], les agents anioniques [esters de l'acide sulfosuccinique : sulfosuccinates de dialkyle, par exemple le dioctylsulfosuccinate de sodium], les agents cationiques (sels d'ammoniums quaternaires).

Les substances édulcorantes ou modifiant le goût peuvent être notamment le saccharose, le glucose, le xylose, le sorbitol, la saccharine, les saccharinates, les cyclamates, l'aspartame, le glycyrrhizinate d'ammonium, ou encore les acides citrique, ascorbique ou tartrique, ou tout autre substance habituellement utilisée pour la modification du goût dans l'industrie alimentaire ou pharmaceutique et qui soit compatible avec les produits mis en présence.

Toutes ces substances peuvent être additionnées indifféremment au début, au cours ou à la fin de la constitution de la pâte à lyophiliser.

Il est entendu que cette nouvelle forme unitaire, solide peut s'appliquer à l'administration de toutes sortes de substances (qu'il s'agisse de substances présentant des problèmes de surfusion ou non) et plus spécialement aux principes actifs pharmaceutiques utilisables par voie orale et destinés aussi bien à la médecine humaine qu'à la médecine vétérinaire. Elle s'applique également aux agents de nutrition, aux agents de diagnostic et aux agents cosmétiques.

A titre d'exemple, parmi les substances pharmaceutiquement actives administrables sous cette forme peuvent être cités les anti-infectieux (spiramycine, pristinamycines, tétracyclines, métronidazole, péfloxacine et dérivés de la famille des quinolones, céfixime, josamycine...), les anti-inflammatoires et anti-rhumatismaux (kétoprofène, l'acide (isobutyl-4 phényl)-2 propionique ...), les analgésiques (aspirine, paracétamol, clométacine...), les tranquillisants (lorazépam, oxazépam, méprobamate, zopiclone et autres dérivés de la famille de cyclopyrrolones, ...), les cardiovasculaires et les vasodilatateurs cérébraux (digoxine, quinacaïnol, propranolol, oxprénolol, vincamine, nicergoline...), les protecteurs cérébraux (gangliosides par exemples), les antispasmodiques et antisécrétoires, les antiasthmatiques, les agents thérapeutiques des maladies du tractus gastro-intestinal, les protecteurs hépatiques, les hormones, les contraceptifs, les médicaments destinés au traitement des allergies, les vaccins, les vitamines ou encore des peptides, des polypeptides ou des protéines.

Parmi les agents de nutrition peuvent être cités notamment des acides aminés, par exemple la méthionine, la lysine, le lysinate de carnitine...

La forme unitaire, solide s'applique également aux agents de diagnostic in vitro, par exemple l'acridine orange (marqueur de phagocytose), ou aux agents de diagnostic in vivo.

Lorsque la nouvelle forme unitaire, solide s'applique aux agents cosmétiques, le principe actif est notamment choisi parmi les substances modifiant l'haleine comme par exemple le menthol et les essences de menthe ou d'eucalyptus.

La quantité du principe actif introduit est variable en fonction de sa nature mais il est bien entendu que cette nouvelle forme solide peut permettre de préparer des doses unitaires à forte teneur en principe actif, permettant ainsi de diminuer la multiplicité des prises compte tenu de la libération contrôlée de ce dernier. D'une manière générale, la quantité de principe actif peut aller jusqu'à 95 % en poids par rapport à la matière sèche.

La nouvelle forme solide selon l'invention présente ainsi l'avantage d'allier une forme primaire unitaire à désagrégation instantanée en milieu aqueux, à une forme secondaire à dissolution contrôlée constituée d'un système particulaire.

La forme primaire permet un emploi plus facile, évite notamment l'agglomération des particules et présente surtout l'avantage de contenir une quantité prédéterminée de principe actif.

La forme secondaire contrôle la libération du principe actif au contact des milieux aqueux. Elle s'adresse tout particulièrement aux substances instables en solution. Par exemple lorsque le principe actif est destiné à l'administration par voie orale, la forme de particules permet de contrôler la mise à disposition du principe actif ; la substance se trouve protégée jusqu'à la zone souhaitée ou jusqu'au moment voulu. La libération de la matière active est alors liée à des facteurs comme le pH, la force ionique, la présence d'une enzyme, la présence d'une flore bactérienne spécifique ou non. Le choix de la dimension des particules permet de prédéterminer la vitesse de libération du principe actif ; par exemple la vitesse de libération en milieu gastrointestinal lorsque le principe actif est administré par voie orale. Cette forme secondaire peut, bien entendu, contenir plusieurs séries de particules soit mélangées les unes aux autres et dans lesquelles les matières actives seront libérées simultanément ou successivement, soit contenues les unes dans les autres et dans lesquelles les matières actives seront libérées successivement. La forme secondaire permet également le masquage du goût de matières actives, par exemple de produits amers.

La forme unitaire, solide selon l'invention lorsqu'elle s'applique aux formulations pharmaceutiques est de ce fait tout spécialement indiquée pour l'administration orale, mais elle peut être également utilisée pour l'administration par voie rectale ou vaginale.

Compte tenu des avantages qu'elle présente, la forme unitaire, solide selon l'invention est tout spécialement indiquée pour les formulations pharmaceutiques orales destinées à la pédiatrie ou à gériatrie, pour les formulations à bioadhésion buccale comme par exemple les systèmes de rafraichissement de l'haleine..., pour les formulations à vectorisation colonique ... ou encore en médecine vétérinaire, dans le cas des adjuvants d'alimentation ou dans le cas des diagnostics médicaux.

Les exemples suivants, illustrent la présente invention.

EXEMPLE 1

* Formule unitaire:

| | | |
|---|---|---|
| . Perles dosées à 50% de kétoprofène | 200,00 | mg |
| . Gomme xanthane | 0,75 | mg |
| . Dioctylsulfosuccinate de sodium | 0,35 | mg |
| . Dextrane 70 | 30,00 | mg |
| . Mannitol | 518,90 | mg |
| ---------------------- | --------- | |
| . Poids unitaire sec | 750,00 | mg |

Composition des perles: 50% Précirol® + 50% kétoprofène

* Mode opératoire:

- Les perles de kétoprofène sont mélangées à sec avec le mannitol dans un mélangeur planétaire.
- La gomme xanthane est dispersée dans de l'eau purifiée (450,00 mg par dose unitaire), puis le dextrane 70 et le dioctylsulfosuccinate de sodium sont dissous. La solution ainsi obtenue est ajoutée au mélange sec. Le mélange pateux est malaxé jusqu'à obtenir une pâte homogène.
- La pâte obtenue est divisée dans des alvéoles de chlorure de polyvinyle de 1,2 cm à raison de 1,2 g par dose unitaire de façon à obtenir 100 mg de kétoprofène par dose.

La préparation est ensuite congelée à une température inférieure à -30°C puis lyophilisée de façon à éliminer l'eau de la préparation.

Les lyophilisats obtenus sont ensuite conditionnés par thermocellage d'une feuille d'aluminium de 20$\mu$m d'épaisseur.

Les lyocs ainsi obtenus se désagrègent dans l'eau en 1 minute environ.

Préparation des perles de kétoprofène:

On prépare des perles contenant 50% de kétoprofène et 50% de Précirol®.

Conditions opératoires:
- Diamètre de la buse: 0,3 mm
- Fréquence des vibrations: 4090 Hz
- Température de la buse: 95°C
- Diamètre moyen des perles: 600 $\mu$m

Le kétoprofène et le Précirol® sont fondus dans un réacteur agité puis transférés dans un ou les deux pots.

L'air de refroidissement est admis au débit de 1,5 à 1,8 m/s.

Le lit fluidisé est mis en service.

Lorsque la température de sortie de l'air est en régime, la vanne $V_1$ ou $V_2$ admet l'azote à la pression choisie et le liquide est transféré dans la buse.

Le réglage de la fréquence est fait au moyen d'un générateur en observant visuellement les perles avec un stroboscope (si nécessaire, l'air de refroidissement est aspiré à travers un lit de carboglace pour qu'il ait une température de 4 à 12°C avant d'entrer dans la tour).

Les perles sont récupérées dans le bas de la tour, elles présentent une dureté suffisante pour être mises en forme pharmaceutique.

Exemple 2

En opérant comme décrit précédemment dans l'exemple 1, mais à partir des constituants suivants:

| . Perles dosées à 50% de kétoprofène | 200,00 mg |
| . Gomme xanthane | 0,75 mg |
| . Dioctylsulfosuccinate de sodium | 0,35 mg |
| . Dextrane 70 | 30,00 mg |
| . Lactose | 518,9  mg |
| ----------------- | --------- |
| Poids unitaire sec | 750,00 mg. |

Les lyophilisats obtenus se désagrègent dans l'eau en 1 minute environ.
Les perles de kétoprofène sont préparées comme décrit précédemment à l'exemple 1.

Exemple 3

En opérant comme décrit dans l'exemple 1 mais à partir des constituants suivants:

| . Perles dosées à 50% de kétoprofène | 200,00 mg |
| . Gomme xanthane | 0,75 mg |
| . Dioctylsulfosuccinate de sodium | 0,35 mg |
| . Dextrane 70 | 50,00 mg |
| . Arôme Menthe | 3,50 mg |
| . Saccharinate de sodium | 2,00 mg |
| . Mannitol | 493,40 mg |
| ----------------- | --------- |
| Poids unitaire sec | 750,00 mg |

L'arôme menthe et le saccharinate de sodium apportent un goût agréable à la préparation qui peut être facilement administrée sans désagrégation préalable dans un verre d'eau.
Les perles de kétoprofène sont préparées comme décrit précédemment à l'exemple 1.

**Revendications**

**Revendications pour les Etats contractants suivants : AT, BE, CH, DE, DK, FR, GB, IT, LI, LU, NL, SE**

1.  Une forme lyophilisée, unitaire, solide et poreuse susceptible d'être obtenue par
    1) préparation d'un mélange de perles par prilling à contre courant du mélange fondu d'une quantité prédéterminée d'un ou plusieurs principes actifs, avec un ou plusieurs excipients choisis parmi des additifs qui permettent d'induire la cristallisation, et éventuellement des polymères solubles ou dispersibles, des additifs minéraux et/ou un agent tensioactif,
    2) incorporation du mélange obtenu dans une pâte destinée à être lyophilisée et contenant
       a) au moins une substance choisie parmi
          - des matières épaississantes et structurantes servant de support et
          - des ballasts,
       b) un ou plusieurs agents stabilisants empêchant la décantation et/ou la remontée en surface des perles dans le mélange,
       c) éventuellement un ou plusieurs autres principes actifs ou mélanges de particules contenant un principe actif,

d) une quantité d'eau convenable de manière à ajuster la viscosité de la composition,

3) lyophilisation de la pâte obtenue, éventuellement après répartition dans des alvéoles de forme et de dimension prédéterminées,

4) éventuellement division du lyophilisat en doses unitaires de forme et de volume prédéterminés.

2. Une forme lyophilisée, unitaire, solide et poreuse selon la revendication 1, caractérisée en ce qu'elle s'applique à une administration par voie orale.

3. Une forme lyophilisée, unitaire, solide et poreuse selon la revendication 1, caractérisée en ce qu'elle s'applique à une administration par voie rectale ou vaginale.

4. Une forme lyophilisée, unitaire, solide et poreuse selon la revendication 1, caractérisée en ce que le principe actif est une substance thérapeutiquement active.

5. Une forme lyophilisée, unitaire, solide et poreuse selon la revendication 1, caractérisée en ce que le principe actif est un agent de nutrition, un agent de diagnostic ou un agent cosmétique.

6. Une forme lyophilisée, unitaire, solide et poreuse selon la revendication 1, caractérisée en ce qu'elle s'applique au domaine vétérinaire.

7. Un procédé de préparation d'une forme lyophilisée, unitaire, solide et poreuse caractérisé en ce que l'on

1) prépare un mélange de perles par prilling à contre courant du mélange fondu d'une quantité prédéterminée d'un ou plusieurs principes actifs, avec un ou plusieurs excipients choisis parmi des additifs qui permettent d'induire la cristallisation, et éventuellement des polymères solubles ou dispersibles, des additifs minéraux et/ou un agent tensioactif,

2) incorpore le mélange obtenu dans une pâte destinée à être lyophilisée et contenant

    a) au moins une substance choisie parmi

      - des matières épaississantes et structurantes servant de support et

      - des ballasts,

    b) un ou plusieurs agents stabilisants empêchant la décantation et/ou la remontée en surface des perles dans le mélange,

    c) éventuellement un ou plusieurs autres principes actifs ou mélanges de perles contenant un principe actif,

    d) une quantité d'eau convenable de manière à ajuster la viscosité de la composition,

3) lyophilise la pâte obtenue, éventuellement après l'avoir répartie dans des alvéoles de forme et de dimension prédéterminées,

4) éventuellement divise le lyophilisat en doses unitaires de forme et de volume prédéterminés.

**Revendication pour les Etats contractants suivants : GR, ES**

1. Procédé de préparation d'une forme lyophilisée, unitaire, solide et poreuse caractérisé en ce que l'on

1) prépare un mélange de perles par prilling à contre courant du mélange fondu d'une quantité prédéterminée d'un ou plusieurs principes actifs, avec un ou plusieurs excipients choisis parmi des additifs qui permettent d'induire la cristallisation, et éventuellement des polymères solubles ou dispersibles, des additifs minéraux et/ou un agent tensioactif,

2) incorpore le mélange obtenu dans une pâte destinée à être lyophilisée et contenant

    a) au moins une substance choisie parmi

      - des matières épaississantes et structurantes servant de support et

      - des ballasts,

    b) un ou plusieurs agents stabilisants empêchant la décantation et/ou la remontée en surface des perles dans le mélange,

    c) éventuellement un ou plusieurs autres principes actifs ou mélanges de perles contenant un principe actif,

    d) une quantité d'eau convenable de manière à ajuster la viscosité de la composition,

3) lyophilise la pâte obtenue, éventuellement après l'avoir répartie dans des alvéoles de forme et de dimension prédéterminées,

4) éventuellement divise le lyophilisat en doses unitaires de forme et de volume prédéterminés.

EP 0 438 358 B1

**Patentansprüche**

**Patentansprüche für folgende Vertragsstaaten : AT, BE, CH, DE, DK, FR, GB, IT, LI, LU, NL, SE**

1. Feste und poröse, lyophilisierte Einzeldosisform, erhältlich durch
   1) Herstellung eines Gemisches aus Perlen durch Gegenstromgranulieren eines geschmolzenen Gemisches einer vorbestimmten Menge eines oder mehrerer Wirkstoffs/Wirkstoffe mit einem oder mehreren Exzipiens/Exzipientien, ausgewählt unter Additiven, die die Kristallisation induzieren können, und gegebenenfalls löslichen oder dispergierbaren Polymeren, mineralischen Additiven und/oder einem grenzflächenaktiven Mittel,
   2) Einarbeiten des so erhaltenen Gemisches in eine Masse, die lyophilisiert werden soll und
      a) mindestens eine Substanz, ausgewählt aus
         - verdickenden und strukturgebenden Mitteln, die als Träger dienen,
         - Füllstoffen,
      b) einen oder mehrere Stabilisator(en), die das Absetzen der Perlen oder ihr Aufsteigen zur Oberfläche in dem Gemisch verhindern,
      c) gegebenenfalls einen oder mehrere andere Wirkstoff(e), oder Gemische von Teilchen, die einen Wirkstoff enthalten,
      d) eine geeignete Menge Wasser, um die Viskosität des Gemisches einzustellen,
         enthält,
   3) Lyophilisation der so erhaltenen Masse, gegebenenfalls nach Verteilung in Zellen mit vorbestimmter Form und vorbestimmter Größe,
   4) gegebenenfalls Aufteilung des Lyophilisats in Einzeldosen mit vorbestimmter Form und vorbestimmtem Volumen.

2. Feste und poröse, lyophilisierte Einzeldosisform nach Anspruch 1, **dadurch gekennzeichnet**, daß sie oral verabreicht wird.

3. Feste und poröse, lyophilisierte Einzeldosisform nach Anspruch 1, **dadurch gekennzeichnet**, daß sie rektal oder vaginal verabreicht wird.

4. Feste und poröse, lyophilisierte Einzeldosisform nach Anspruch 1, **dadurch gekennzeichnet**, daß der Wirkstoff eine therapeutisch wirksame Substanz ist.

5. Feste und poröse, lyophilisierte Einzeldosisform nach Anspruch 1, **dadurch gekennzeichnet**, daß der Wirkstoff ein Nährmittel, ein diagnostisches Mittel oder ein kosmetisches Mittel ist.

6. Feste und poröse, lyophilisierte Einzeldosisform nach Anspruch 1, **dadurch gekennzeichnet**, daß sie auf dem ve terinärmedizinischen Gebiet angewendet wird.

7. Verfahren zur Herstellung einer festen und porösen, lyophilisierten Einzeldosisform, **dadurch gekennzeichnet**, daß man
   1) ein Gemisch aus Perlen durch Gegenstromgranulieren eines geschmolzenen Gemisches einer vorbestimmten Menge eines oder mehrerer Wirkstoffs/Wirkstoffe mit einem oder mehreren Exzipiens/Exzipientien, ausgewählt unter Additiven, die die Kristallisation induzieren können, und gegebenenfalls löslichen oder dispergierbaren Polymeren, mineralischen Additiven und/oder einem grenzflächenaktiven Mittel, herstellt,
   2) das so erhaltene Gemisch in eine Masse, die lyophilisiert werden soll und
      a) mindestens eine Substanz, ausgewählt aus
         - verdickenden und strukturgebenden Mitteln, die als Träger dienen,
         - Füllstoffen,
      b) einen oder mehrere Stabilisator(en), die das Absetzen der Perlen oder ihr Aufsteigen zur Oberfläche in dem Gemisch verhindern,
      c) gegebenenfalls einen oder mehrere andere Wirkstoff(e), oder Gemische von Teilchen, die einen Wirkstoff enthalten,
      d) eine geeignete Menge Wasser, um die Viskosität des Gemisches einzustellen,
         enthält,
   einarbeitet,
   3) die so erhaltene Masse, gegebenenfalls nach Verteilung in Zellen mit vorbestimmter Form und vorbestimmter Größe, lyophilisiert,

4) gegebenenfalls das Lyophilisat in Einzeldosen mit vorbestimmter Form und vorbestimmtem Volumen aufteilt.

**Patentanspruch für folgende Vertragsstaaten : ES, GR**

1. Verfahren zur Herstellung einer festen und porösen lyophilisierten Einzeldosisform, **dadurch gekennzeichnet**, daß man

   1) ein Gemisch aus Perlen durch Gegenstromgranulieren eines geschmolzenen Gemisches einer vorbestimmten Menge eines oder mehrerer Wirkstoffs/Wirkstoffe mit einem oder mehreren Exzipiens/Exzipientien, ausgewählt unter Additiven, die die Kristallisation induzieren können, und gegebenenfalls löslichen oder dispergierbaren Polymeren, mineralischen Additiven und/oder einem grenzflächenaktiven Mittel, herstellt

   2) das so erhaltene Gemisch in eine Masse, die lyophilisiert werden soll und

      a) mindestens eine Substanz, ausgewählt aus

        - verdickenden und strukturgebenden Mitteln, die als Träger dienen,

        - Füllstoffen,

      b) einen oder mehrere Stabilisator(en), die das Absetzen der Perlen oder ihr Aufsteigen zur Oberfläche in dem Gemisch verhindern,

      c) gegebenenfalls einen oder mehrere andere Wirkstoff(e), oder Gemische von Teilchen, die einen Wirkstoff enthalten,

      d) eine geeignete Menge Wasser, um die Viskosität des Gemisches einzustellen,

        enthält,

   einarbeitet,

   3) die so erhaltene Masse, gegebenenfalls nach Verteilung in Zellen mit vorbestimmter Form und vorbestimmter Größe, lyophilisiert,

   4) gegebenenfalls das Lyophilisat in Einzeldosen mit vorbestimmter Form und vorbestimmtem Volumen aufteilt.

## Claims

**Claims for the following Contracting States : AT, BE, CH, DE, DK, FR, GB, IT, LI, LU, NL, SE**

1. A solid and porous, unitary, freeze-dried Form capable of being obtained by

   1) preparation of a mixture of prills by countercurrent prilling of the molten mixture of a predetermined quantity of one or a number of active principles, with one or a number of excipients chosen from additives which make it possible to induce crystallization, and optionally of soluble or dispersible polymers, of inorganic additives and/or a surface-active agent,

   2) incorporation of the mixture obtained into a paste intended to be freeze-dried and containing

      a) at least one substance chosen from

        - thickening and structuring materials acting as carrier and

        - ballasts,

      b) one or a number of stabilizing agents preventing sedimentation and/or return to the surface of the prills in the mixture,

      c) optionally one or a number of other active principles or mixtures of particles containing an active principle,

      d) a suitable quantity of water so as to adjust the viscosity of the composition,

   3) freeze-drying of the paste obtained, optionally after distribution into cells of predetermined shape and dimension,

   4) optionally division of the freeze-dried material into unitary doses of predetermined shape and volume.

2. A solid and porous, unitary, freeze-dried form according to Claim 1, characterized in that it applies to an administration by oral route.

3. A solid and porous, unitary, freeze-dried form according to Claim 1, characterized in that it applies to an administration by rectal or vaginal route.

4. A solid and porous, unitary, freeze-dried form according to Claim 1, characterized in that the active principle is a therapeutically active substance.

5. A solid and porous, unitary, freeze-dried form according to Claim 1, characterized in that the active principle is a nutrient agent, a diagnostic agent or a cosmetic agent.

6. A solid and porous, unitary, freeze-dried form according to Claim 1, characterized in that it applies to the veterinary field.

7. Process for the preparation of a solid and porous, unitary, freeze-dried form, characterized in that
1) a mixture of prills is prepared by countercurrent prilling of the molten mixture of a predetermined quantity of one or a number of active principles, with one or a number of excipients chosen from additives which make it possible to induce crystallization, and optionally of soluble or dispersible polymers, of inorganic additives and/or a surface-active agent,
2) the mixture obtained is incorporated into a paste intended to be freeze-dried and containing
   a) at least one substance chosen from
     - thickening and structuring materials acting as carrier and
     - ballasts,
   b) or a number of stabilizing agents preventing sedimentation and/or return to the surface of the prills in the mixture,
   c) optionally one or a number of other active principles or mixtures of prills containing an active principle,
   d) a suitable quantity of water so as to adjust the viscosity of the composition,
3) the paste obtained is freeze-dried, optionally after it has been distributed into cells of predetermined shape and dimension,
4) the freeze-dried material is optionally divided into unitary doses of predetermined shape and volume.

**Claim for the following Contracting States : GR, ES**

1. Process for the preparation of a solid and porous, unitary, freeze-dried form, characterized in that
1) a mixture of prills is prepared by countercurrent prilling of the molten mixture of a predetermined quantity of one or a number of active principles, with one or a number of excipients chosen from additives which make it possible to induce crystallization, and optionally of soluble or dispersible polymers, of inorganic additives and/or a surface-active agent,
2) the mixture obtained is incorporated into a paste intended to be freeze-dried and containing
   a) at least one substance chosen from
     - thickening and structuring materials acting as carrier and
     - ballasts,
   b) or a number of stabilizing agents preventing sedimentation and/or return to the surface of the prills in the mixture,
   c) optionally one or a number of other active principles or mixtures of prills containing an active principle,
   d) a suitable quantity of water so as to adjust the viscosity of the composition,
3) the paste obtained is freeze-dried, optionally after it has been distributed into cells of predetermined shape and dimension,
4) the freeze-dried material is optionally divided into unitary doses of predetermined shape and volume.

FIGURE 1